(19) Europäisches Patentamt

European Patent Office

Office européen des brevets

(11) **EP 0 609 153 B1**

(12) **FASCICULE DE BREVET EUROPEEN**

(45) Date de publication et mention
de la délivrance du brevet:
**09.09.1998 Bulletin 1998/37**

(51) Int. Cl.$^6$: **B01J 19/12**, B01J 10/00

(21) Numéro de dépôt: **94400185.8**

(22) Date de dépôt: **28.01.1994**

(54) **Nouveau procédé photochimique gaz/liquide d'halogénation d'alkylbenzènes**

Photochemisches Verfahren zur Gas-Flüssigkeit-Halogenierung von Alkylbenzolen

Photochemical gas/liquid process for the halogenation of alkylbenzenes

(84) Etats contractants désignés:
**AT BE CH DE DK ES FR GB GR IE IT LI LU NL PT SE**

(30) Priorité: **28.01.1993 FR 9300863**

(43) Date de publication de la demande:
**03.08.1994 Bulletin 1994/31**

(73) Titulaire: **ELF ATOCHEM S.A.**
**92800 Puteaux, Hauts-de-Seine (FR)**

(72) Inventeur: **Desire, Gérard**
**F-92300 Lens (FR)**

(74) Mandataire:
**Hirsch, Marc-Roger et al**
**Cabinet Hirsch**
**34 rue de Bassano**
**75008 Paris (FR)**

(56) Documents cités:
EP-A- 0 091 358          EP-A- 0 111 253
FR-A- 1 416 096          FR-A- 2 156 911

- PATENT ABSTRACTS OF JAPAN vol. 11, no. 013 (C-397)14 Janvier 1987 & JP-A-61 187 930 (ISHIKAWAJIMA HARIMA HEAVY IND. CO. LTD.) 21 Août 1986 & CHEMICAL ABSTRACTS, vol. 106, no. 2, 12 Janvier 1987, Columbus, Ohio, US; abstract no. 6807x, 'PHOTOCHEMICAL REACTORS'
- PATENT ABSTRACTS OF JAPAN vol. 13, no. 423 (C-638)20 Septembre 1989 & JP-A-11 060 476 (NIPPON SHARYO SEIZO KAISHA LTD.) 23 Juin 1989
- CHEMIE. INGENIEUR. TECHNIK vol. 56, no. 1 , Janvier 1984 , WEINHEIM DE pages 64 - 65 P.WEILAND 'Einfluss des Kernrohrdurchmessers auf das Betriebsverhalten von Airlift-Schlaufenreaktoren'
- Ullmann's Encyclopedia of Industrial Chemistry (1986) pages 355-363, VCH Verlagsgesellschaft mbH, Weinheim, DE

**Description**

La présente invention a pour objet un nouveau procédé photochimique gaz/liquide d'halogénation d'alkylbenzènes, en particulier chloration de toluène.

La chloration, ou halogénation au sens large, photochimique de dérivés hydrocarbonés est largement utilisée actuellement. Cependant, comme il s'agit de réactions de substitution d'atome d'hydrogène par des atomes de chlore, il se pose de nombreux problèmes de sélectivité. Un exemple représentatif d'une chloration photochimique est la chloration d'alkylbenzène, en particulier le toluène.

La chloration photochimique de toluène conduit principalement à la formation de chlorure de benzyle, de chlorure de benzylidène et de phénylchloroforme.

On sait également qu'à côté de cette chloration principale du groupement méthyle de la molécule de toluène, il se forme toujours des petites quantités indésirables de dérivés chlorés sur le noyau aromatique, à savoir les chlorotoluènes et leurs homologues les chlorochlorures de benzyle, les chlorochlorures de benzylidène et les chlorophénylchloroformes.

On sait en outre que la proportion de dérivés chlorés sur le noyau aromatique augmente sensiblement avec le taux de chloration du mélange. De 0,1 à 0,5% quand on se limite au chlorure de benzyle, la proportion de dérivés chlorés sur le noyau aromatique peut atteindre, voire dépasser 5% quand on veut obtenir la chloration totale du groupement méthyle de façon à produire essentiellement le phénylchloroforme.

Pour cette raison, il est difficile d'obtenir du phénylchloroforme de haute pureté par chloration directe sans recourir à la distillation du mélange réactionnel. Certains brevets préconisent donc de faire une chloration incomplète pour limiter la formation de dérivés nucléaires et de distiller ensuite les mélanges obtenus moyennant une augmentation sensible du prix de revient.

De nombreux réacteurs photochimiques ont été développés, certains à usage spécifique. Ainsi, JP-A-61 87930 a pour objet un réacteur de chloration de benzène, réaction de chloration ne présentant pas de façon critique les problèmes de sélectivité énumérés ci-dessus pour le toluène. Ce réacteur est un réacteur particulier avec une convection de fluide. Ce réacteur cylindrique vertical possède une paroi cylindrique interne constituée d'un miroir. La circulation interne du fluide se fait par un flux ascendant à l'intérieur du cylindre et un flux descendant entre l'enveloppe externe et cette paroi interne. L'alimentation en gaz se fait par le fond du réacteur et la source lumineuse est au sein du réacteur.

Les documents "Reaktionsapparate fur Gas-Flüssig-Reaktionen", p. 372, et "Chemischtechnisches Lexiken" décrivent des réacteurs, de type gazosiphon, et/ou possédant un courant d'entraînement injecté en pied. L'exemple de mise en oeuvre est l'oxydation du n-butane.

Aucun document n'enseigne ni ne suggère le procédé selon la présente invention.

Ainsi, la présente invention fournit un procédé photochimique gaz/liquide qui permet une sélectivité élevée, ainsi que d'autres avantages.

- la figure 1      représente le schéma général d'un réacteur pour la mise en oeuvre du présent procédé;
- la figure 2      représente le schéma d'un mode de réalisation d'un réacteur pour la mise en oeuvre du présent procédé;
- la figure 3      représente le schéma d'un second mode de réalisation d'un réacteur pour la mise en oeuvre du présent procédé;
- la figure 4      représente une variante d'un réacteur pour la mise en oeuvre du présent procédé;
- la figure 5      représente une autre variante d'un réacteur pour la mise en oeuvre du présent procédé;
- la figure 6      représente un distributeur;
- la figure 7      représente une variante d'un réacteur pour la mise en oeuvre du présent procédé;
- la figure 8      représente un exemple d'une installation ou ligne de production comprenant plusieurs réacteurs;
- la figure 9      représente une variante d'un mode de réalisation de l'invention.

La présente invention a donc pour objet un procédé d'halogénation sélective d'alkylbenzènes sur la (les) chaîne(s) alkyle(s) caractérisé en ce qu'il est mis en oeuvre dans un réacteur gaz/liquide photochimique (1) à symétrie axiale, comprenant:

- une enceinte;
- une source centrale de rayonnement;
- au moins un distributeur de gaz, en pied dudit réacteur;
- une virole disposée concentriquement entre la source de rayonnement et l'enceinte, ladite virole permettant une recirculation interne du liquide réactionnel, selon un mouvement ascendant dans l'espace situé entre la source et la virole, et selon un mouvement descendant dans l'espace situé entre l'enceinte et la virole.

De préférence, le procédé est un procédé de chloration du toluène.

Le réacteur utilisé dans les procédés selon l'invention permet de définir trois zones à l'intérieur de celui-ci, disposées concentriquement:

- une zone centrale où est installée la source de rayonnement;
- une zone réactionnelle annulaire autour de la zone centrale comprenant, à la partie inférieure, un système de distribution de gaz à orifices calibrés permettant d'imposer la taille des bulles de gaz. La zone réactionnelle, i.e. dans l'espace situé entre la source et la virole, tire son originalité de la recirculation ascendante contrôlée du liquide dans celle-ci le long de la source;
- une zone périphérique à circulation descendante contrôlée du liquide échangé avec la zone réactionnelle. Cette zone permet en outre les échanges thermiques nécessaires au maintien de températures appropriées par l'intermédiaire des surfaces d'échanges thermique ( chauffage ou le plus souvent refroidissement dont est équipée la paroi externe du réacteur).

La présente invention permet aussi d'atteindre les buts suivants:

. la section de la zone réactionnelle d'un réacteur à recirculation interne est environ trois fois inférieure à celle d'un réacteur classique sans recirculation pour un même débit de gaz;
. la hauteur de liquide que le courant gazeux doit traverser est environ deux à trois fois inférieure, dans un réacteur à recirculation du liquide le long de la source de rayonnement, à celle qu'il est nécessaire de traverser dans un réacteur classique pour atteindre une fixation équivalente du chlore;
. la recirculation du liquide le long de la source de rayonnement permet une utilisation beaucoup plus efficace de celle-ci. Il est possible de ce fait de réduire le nombre de la puissance des lampes. Pour cette raison, il est possible de loger les lampes dans un seul tube central même pour des réacteurs de grande capacité;
. la présence d'une virole intermédiaire proche de la paroi du réacteur permet d'augmenter considérablement, jusqu'à trois fois, le coefficient global d'échange calorifique. Cette augmentation du coefficient d'échange est particulièrement utile dans la conception des réacteurs de grande capacité;
. la combinaison des avantages ci-dessus permet de concevoir des réacteurs compacts beaucoup plus petits que les réacteurs classiques à capacité égale de production ou d'atteindre des capacités unitaires très élevées absolument inaccessibles au moyen de réacteurs classiques sans recirculation.

Selon un mode de réalisation, la virole s'étend jusqu'au niveau du distributeur ou jusqu'à un niveau intermédiaire situé entre le distributeur et le pied de l'enceinte.

Selon un autre mode de réalisation, la virole s'étend jusqu'au pied de l'enceinte et comporte une série d'ouvertures situées à un niveau intermédiaire entre le distributeur et le pied de l'enceinte.

Cette virole peut avoir toute configuration, tant qu' elle remplit sa fonction de recirculation. Notamment, elle peut être l'image, par une homothétie d'ordre inférieur à 1, de l'enceinte du réacteur. Cette virole peut présenter, en coupe, une droite ou un segment à l'extrémité inférieure ou supérieure ou aux deux extrémités duquel peut se trouver un ou plusieurs autres segments ou courbes de droite présentant un angle avec le segment principal. La virole peut aussi être constituée de sous-viroles, disposées horizontalement en ligne et constituer ainsi une virole calibrée, ou alternativement décalées les unes par rapport aux autres et ainsi, en coupe, se présenter sous la forme de segments de droite, disposés en ligne mais présentant un angle avec la direction générale de la virole. Cette virole peut encore consister en des sous-viroles de disposition générale verticale, mais présentant des interstices plus ou moins grands entre chacune d'entre elles. Elles peuvent en outre être décalées les unes par rapport aux autres selon le rayon du réacteur. Enfin, la virole peut agir à la façon d'un miroir pour réfléchir vers le centre du réacteur le rayonnement émis par la source.

Toute disposition de virole est appropriée si celle-ci remplit la fonction de recirculation. Ainsi, ce terme de virole, tel qu'utilisé dans la présente invention, doit-il être compris comme "moyen assurant la recirculation interne du liquide".

Selon un mode de réalisation, la virole comporte sur une partie ou toute sa longueur des fentes calibrées.

La recirculation interne, caractéristique du réacteur pour la mise en oeuvre du présent procédé, commence de préférence sous le distributeur, et le courant de liquide traverse donc l'espace délimité par le distributeur.

Ainsi, selon un mode de réalisation, le procédé est mis en oeuvre dans un réacteur dans lequel la recirculation interne commence à un niveau situé sous le distributeur (4) dans une région située en pied de l'enceinte (2).

La source centrale de rayonnement peut être disposée au sein du réacteur selon plusieurs possibilités.

Selon une variante de la présente invention, la source traverse le réacteur de part en part.

Selon une autre variante de la présente invention, la source traverse le couvercle du réacteur uniquement.

Cette source centrale de rayonnement est de préférence constituée de un ou plusieurs tube(s) transparent(s) disposé(s) selon une symétrie axiale. Avantageusement, le tube est unique.

Le distributeur de gaz est tout distributeur approprié, tel qu'une grille, des tores percés concentriques ou toute autre variante aisément accessible à l'homme de l'art.

De préférence, le ou les distributeur(s) est(sont) constitué(s) de un ou plusieurs tore(s) concentrique(s) percés de trous calibrés sur leurs moitiés supérieures.

Selon un mode de réalisation, le réacteur pour la mise en oeuvre du présent procédé comprend un unique distributeur en pied de l'enceinte.

Selon un autre mode de réalisation, le réacteur pour la mise en oeuvre du présent procédé comprend au moins deux distributeurs, situés à des niveaux différents dans le réacteur.

Lors de la mise en oeuvre du réacteur, les conditions opératoires de température, pression, et autres, utilisées pour la réaction considérée sont celles habituellement utilisées.

De préférence, la vitesse du liquide selon le mouvement ascendant dans l'espace situé entre la source et la virole est comprise entre 0,1 et 0,8 m/s, de préférence comprise entre 0,25 et 0,60 m/s.

La vitesse du liquide sera notée U tandis que la vitesse du gaz par rapport au liquide sera notée V. La vitesse du gaz par rapport au réacteur est U + V. Selon un mode de réalisation, la vitesse du gaz U + V est comprise entre 0,3 et 1,1 m.s$^{-1}$.

De préférence, la vitesse du liquide selon le mouvement descendant dans l'espace situé entre la virole et l'enceinte est comprise entre 0,5 et 3 m/s, de préférence entre 1 et 2,5 m/s. De préférence, la fraction de volume occupé par la phase gaz représente de 10 à 50%, de préférence de 20 à 40% en volume, du volume compris entre la source et la virole. De préférence, le diamètre des bulles générées par le distributeur est compris entre 3 et 15 mm, et de préférence entre 6 et 10 mm.

Le réacteur selon la présente invention est un réacteur à symétrie axiale, de préférence il s'agit d'un réacteur de révolution. Il peut être cylindrique ou cylindroconique.

Le réacteur, ainsi que sa mise en oeuvre, est décrit maintenant plus en détail, en référence aux figures.

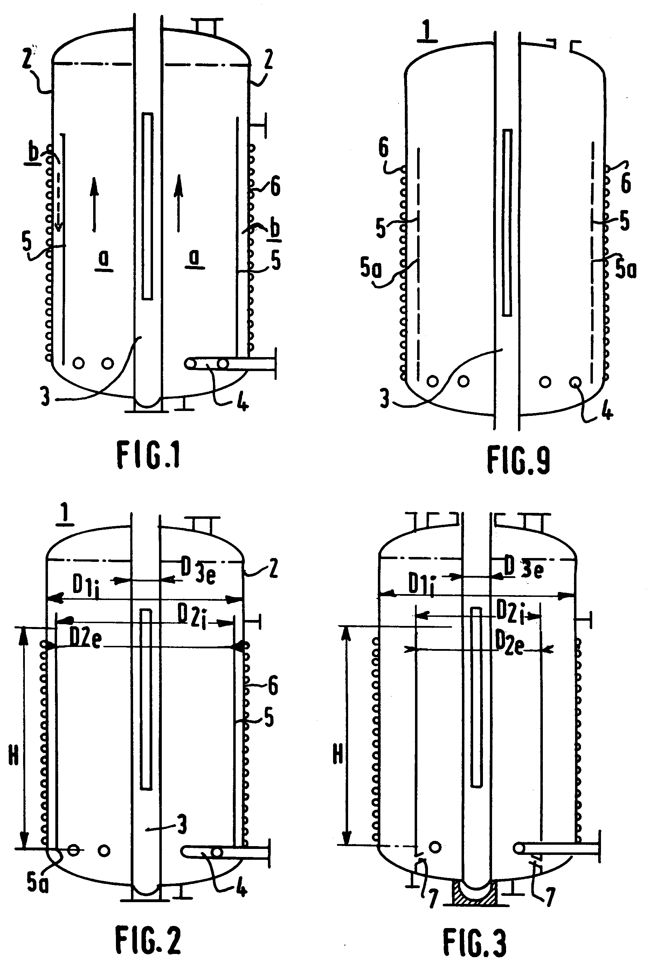

La figure 1 représente le schéma général d'un réacteur selon la présente invention. Ce réacteur 1 est constitué de plusieurs éléments. Ceux-ci sont fabriqués en des matériaux appropriés pour la réaction chimique considérée. Par exemple, dans le cas de la chloration du toluène, les éléments du réacteur sont fabriqués à partir d'alliages non-ferreux, par exemple de nickel ou d'alliages de celui-ci. Le réacteur 1 comprend une enceinte 2, une source centrale de rayonnement 3, classiquement un tube transparent, et un distributeur 4. Les dimensions du réacteur, défini par l'enceinte, sont classiquement: longueur: 1 à 4 m, avantageusement 1,5 à 3 m, diamètre: 0,5 à 2 m, avantageusement 0,7 à 1,5 m. Le ou les tube(s) transparent(s) sont généralement en verre pyrex ou en quartz, ou sont en tout autre matériau transparent dans l'intervalle encadrant la longueur d'onde de rayonnement utilisé. Classiquement, la longueur d'onde correspond à l'ultra-violet (U.V.). Ce tube peut être en un matériau transparent vers la longueur d'onde utilisée, par exemple l'U.V., et sensiblement opaque aux autres longueurs d'onde, afin de limiter la formation de sous-produits ou la dégradation des produits recherchés, qui pourrait éventuellement avoir lieu à ces longueurs d'onde différents de la longueur d'onde de travail. Ce résultat est atteint, par exemple, avec le verre pyrex qui laisse passer l'U.V., c'est-à-dire un rayonnement à environ 250 à 400 nm, mais est sensiblement opaque aux radiations inférieures à 200 nm. Le nombre et la puissance des lampes varient avec la capacité de production recherchée. Le nombre de lampes est généralement compris entre 1 et 10, et le plus souvent entre 2 et 4. La puissance unitaire des lampes varie entre 0,1 kW et 20 kW, plus souvent entre 0,2 et 4 kW. Ces lampes peuvent être disposées autour d'une tige centrale servant de support, ou être disposées dans plusieurs tubes. Ainsi, il est possible d'utiliser soit un émetteur central bi-lampe, soit 3 émetteurs mono-lampe; on tiendra compte de l'influence de la disposition des émetteurs sur les écoulements hydrauliques dans le réacteur. Selon les dimensions du tube, les lampes sont au même niveau ou à des niveaux différents. Les dimensions du ou des tubes sont classiques, et correspondent, en longueur, sensiblement à la longueur du réacteur. Dans les faits, la longueur du tube est telle que celui-ci est en immersion dans substantiellement tout le fluide réactionnel. Le diamètre du tube central, lorsqu'il est unique, est classiquement compris entre 50 et 500 mm, avantageusement 100 à 200 mm. Le réacteur comprend de plus, selon la présente invention, une virole 5 assurant la recirculation. Ainsi, le réacteur cylindrique ou cylindroconique, ayant la disposition selon la présente invention, comprend, de l'axe vers la périphérie:

- un tube central transparent destiné au logement de la source de rayonnement;
- une zone annulaire réactionnelle (a) autour du tube transparent central;
- une zone périphérique (b) autour de la zone réactionnelle dont le rôle est d'assurer une recirculation du liquide de la zone réactionnelle et de faciliter les échanges thermiques.

Dans la zone (a), le sens de recirculation est indiqué par la flèche pleine, tandis que dans la zone (b), le sens de circulation est indiqué par la flèche en pointillé. A la partie supérieure de la zone réactionnelle, le liquide entraîné se déverse par trop-plein, ou surverse, dans la zone périphérique de recyclage et d'échange thermique, tandis que le gaz, constitué du gaz introduit n'ayant pas réagi et éventuellement de gaz produit par la réaction, s'évacue à la partie supérieure du réacteur. Cependant, le gaz n'ayant pas réagi est souvent négligeable compte tenu de l'efficacité des présents réacteurs. Par exemple, ce n'est qu'en fin de chloration, lors de la chloration du groupe méthyle substituant sur le cycle aromatique, que le débit de chlore non-fixé devient négligeable. En fait, on peut considérer que le débit gazeux est majoritairement constitué du gaz introduit et des composés organiques vaporisés, quel que soit le taux de fixation du gaz. Le réacteur comprend aussi une enveloppe externe 6, du type double paroi ou de préférence demi-coquilles, permettant d'assurer les échanges thermiques. L'existence de la virole ou paroi intermédiaire permet d'augmenter considérablement les coefficients de transfert thermique et donc de diminuer nettement les surfaces d'échange, par rapport à celles requises dans un réacteur classique sans recirculation interne.

La virole 5 peut être placée dans le réacteur selon plusieurs possibilités. Les figures 2 et 3 donnent deux modes de réalisation.

La figure 2 est une représentation du mode de réalisation selon lequel la virole intermédiaire s'arrête au niveau du distributeur ou à un niveau entre le distributeur et le fond du réacteur.

Les références numériques sont identiques à celles de la figure 1. Le fluide s'écoule librement vers la zone réactionnelle, sous le(s) distributeur(s) de gaz. La surface S' est la surface délimitée par le diamètre intérieur de l'enceinte, $D_{1i}$, et par le diamètre extérieur de la virole, $D_{2e}$, soit:

$$S' = \frac{\pi}{4} (D_{1i}^{2} - D_{2e}^{2})$$

C'est cette surface S' qui détermine la vitesse et le débit de recirculation, et par là la vitesse d'écoulement dans la zone réactionnelle, de surface S. La surface S est délimitée par le diamètre intérieure de la virole, $D_{2i}$, et le diamètre extérieur du tube transparent, $D_{3e}$, soit:

$$S = \frac{\pi}{4} (D_{2i}^{2} - D_{3e}^{2})$$

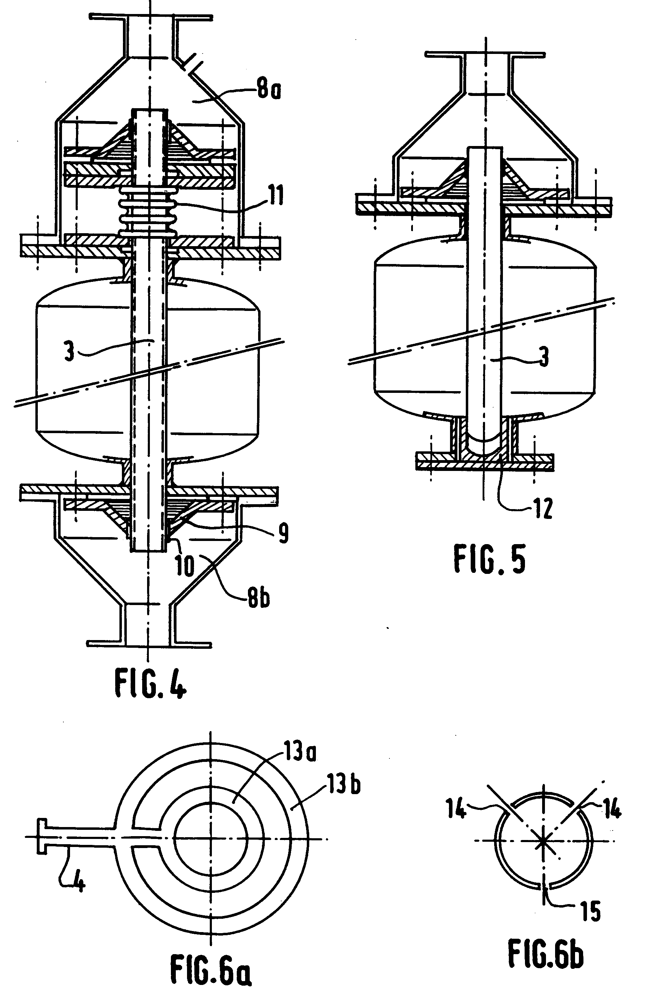

La section S' est calculée pour que la vitesse spécifiée du liquide dans la zone réactionnelle soit comprise entre 0,1 et 0,8 m.s$^{-1}$, et que la fraction de volume occupée par le gaz représente 10 à 50%, de préférence 20 à 40%, du volume de la zone réactionnelle. La vitesse de l'écoulement dans la zone de recirculation entre la virole et l'enceinte est alors généralement comprise entre 0,5 et 3 m.s$^{-1}$. Ce mode de réalisation, où la virole s'arrête au niveau du distributeur ou à un niveau compris entre le distributeur et le pied du réacteur, est recommandé mais n'est pas limité aux cas où la cinétique de réaction est rapide et requiert un échange thermique efficace, rendu possible par la vitesse élevée du liquide dans la zone de recirculation. La figure 2 représente la variante spécifique selon laquelle la virole s'arrête au niveau du distributeur, sur la figure 5a.

La figure 3 est une représentation d'un autre mode de réalisation, dans lequel la virole descend jusqu'au fond du réacteur. Des orifices 7 sont disposées à la partie inférieure de la virole intermédiaire. De préférence, ils sont orientés vers le distributeur de gaz, de façon à assurer un renouvellement permanent de liquide à ce niveau. Cette orientation est représentée sur la figure 3. Ces orifices peuvent être des buses, des trous circulaires, des fentes rectangulaires et autres. Le nombre et la section de ces orifices sont déterminés de façon à régler le rapport débit liquide/débit de gaz, à la valeur choisie. En ajustant ce rapport, on ajuste ainsi la vitesse d'écoulement dans la zone réactionnelle. Dans ce mode de réalisation, la surface S' ou section de la zone périphérique de recirculation ne détermine plus le débit de liquide, et peut être augmentée à volonté. Cette configuration est recommandée, mais non limitée, aux réactions dont la vitesse de réaction est relativement lente et lorsque l'on souhaite conserver une masse de liquide suffisante dans la zone périphérique pour la stabilité de fonctionnement. Les conditions opératoires, en ce qui concerne la vitesse d'écoulement et la fraction de volume occupé par la phase gazeuse, sont similaires à celles selon le mode de réalisation précédent.

La figure 4 représente un mode de réalisation selon lequel le tube, siège de la source de rayonnement, traverse le réacteur de part en part. Le tube est alors un tube ouvert aux deux extrémités. Il peut traverser les tubulures centrales du couvercle 8a et du fond du réacteur 8b, lorsque de telles tubulures sont présentes. Ces tubulures d'amenée de gaz en pied et de soutirage en tête peuvent bien sûr être latérales, comme dans toute variante du réacteur selon la présente invention. Une grille 9 de retenue solidaire de la tubulure du fond de réacteur permet d'empêcher le tube de glisser et sert en même temps d'appui d'une tige centrale destinée au support des lampes. L'étanchéité en fond de réacteur est assurée par un assemblage coulissant comprenant notamment un joint à collerette 10, généralement en PTFE ou tout autre matériau, résistant au milieu réactionnel et une bride de serrage du joint à collerette par l'intermédiaire d'un support souple. L'étanchéité au niveau du couvercle du réacteur s'obtient suivant le même principe mais avec, en outre, un soufflet de dilatation 11 destiné à compenser les variations de dilatation entre le tube transparent et le réacteur. Les tubulures du couvercle et du fond de réacteur sont connectées à un circuit de sorte qu'en cas très improbable de rupture du tube transparent, il n'y a pas à craindre de perte de confinement dans le milieu extérieur. L'ouverture du tube transparent aux deux extrémités permet une circulation contrôlée et pressurisée de gaz inerte à l'intérieur dudit tube transparent.

La figure 5 représente un mode de réalisation selon lequel le tube traverse le couvercle seulement. Dans ce cas, il s'agit d'un tube borgne, c'est-à-dire fermé à la partie inférieure. L'extrémité inférieure arrondie repose sur un bouchon 12, de préférence en PTFE, placé dans la tubulure du fond de réacteur. La forme creuse du bouchon permet au tube transparent de coulisser suffisamment pour absorber les variations de dilatation entre le tube transparent et le réacteur. On évite ainsi le recours au soufflet de dilatation au niveau du couvercle du réacteur.

Les figures 6a et 6b représentent une coupe d'un distributeur selon un mode de réalisation de la présente invention. La figure 6a représente un distributeur. La figure 6b est une coupe de ce distributeur. Ce distributeur est constitué d'un ou plusieurs tores concentriques 13a et 13b selon la taille du réacteur et percés de trous 14 calibrés à la dimension spécifiée, généralement comprise entre 3 et 15 mm et, le plus souvent, entre 6 et 10 mm. Entre les couronnes, on laisse un espace libre qui représente de 40 à 80% de la section de la zone réactionnelle et qui est destiné au passage du liquide recyclé sous le distributeur.

Comme indiqué à la figure 6b, les trous calibrés sont généralement disposés sur la moitié supérieure du tore en deux rangées séparées par un angle au centre variable, mais le plus souvent compris entre 60 et 180°C et, de préférence, de 90 à 135°C pour faciliter l'entraînement des bulles de gaz dans le courant de liquide de recirculation. On prévoit également quelques trous plus petits 15 à la partie inférieure pour assurer l'écoulement du liquide à l'arrêt et au redémarrage. Les bulles de gaz introduites entraînent le liquide recyclé à une vitesse déterminée et dans un rapport phase gaz/phase liquide bien défini optimisé de manière à obtenir la meilleure cinétique de réaction. Habituellement, on dimensionne le réacteur de manière à obtenir une vitesse de circulation du liquide comprise entre 0,1 et 0,8 m/s, de préférence 0,25 et 0,60 m/s, et une fraction de phase gazeuse de 10 à 50%, de préférence 20 à 40%, du volume de la zone réactionnelle.

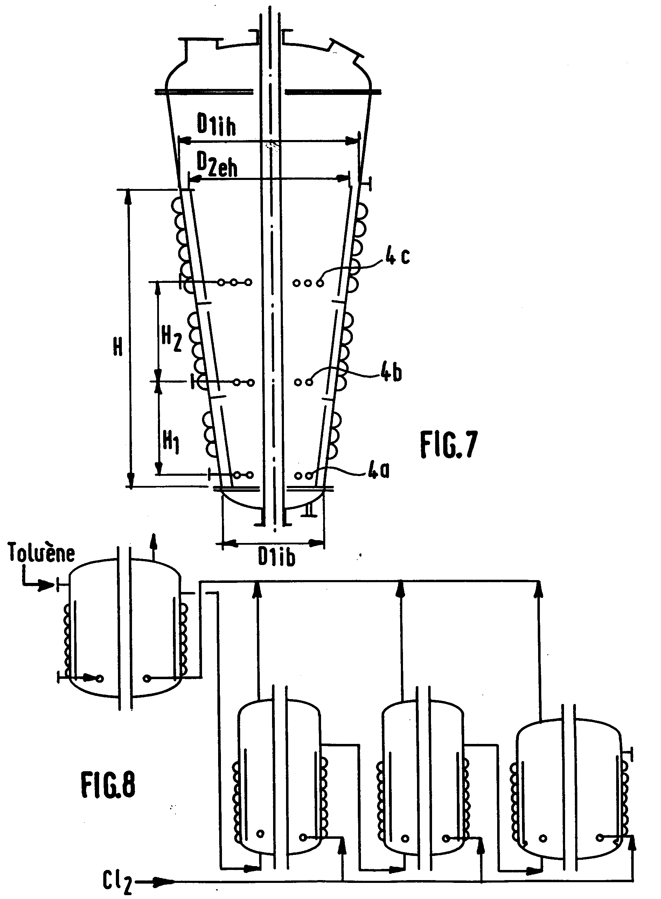

La figure 7 représente une variante de la présente invention, dans laquelle plusieurs distributeurs sont présents et dans laquelle le réacteur est cylindroconique. Trois niveaux de distribution 4a, 4b et 4c sont représentés sur cette figure, aux niveaux 0, $H_1$ et $H_2$. H est la hauteur totale de la zone réactionnelle. Par ailleurs, les dimensions du réacteur cylindroconique sont données par $D_1ib$, diamètre intérieure à la base de l'enceinte, $D_1ih$, diamètre intérieur en hauteur de l'enceinte, et $D_2eh$, diamètre extérieur en hauteur de la virole. En fait, le débit gazeux des distributeurs inférieurs s'ajoutent à celui des distributeurs supérieurs. Ainsi, le réacteur est de forme cylindroconique de façon à maintenir sensiblement les mêmes paramètres d'écoulement à tous les niveaux du réacteur.

La figure 8 représente un schéma de mise en oeuvre de plusieurs réacteurs selon la présente invention. Le mode de fonctionnement est explicité dans l'exemple 2.

La figure 9 représente une variante d'un réacteur selon la présente invention. La virole est une virole possédant des fentes calibrées 5a. Les fentes peuvent n'être présentes que sur une partie seulement de la virole. Ceci permet d'amorcer et d'entretenir la recirculation quel que soit le niveau de remplissage du réacteur. Les autres références numériques sont identiques à celles utilisées pour les figures 1 à 3.

La présente invention fournit donc un nouveau procédé d'halogénation d'alkylbenzènes, mis en oeuvre dans les conditions exposées ci-avant. A titre d'exemple, et de façon non-limitative, on peut citer les réactions suivantes:

- chloration des chaînes latérales des hydrocarbures aromatiques:

  . méthylbenzènes (toluène, xylènes, tri- et polyméthylbenzènes),
  . autres alkylbenzènes; dans ce cas, c'est la chloration sur le carbone voisin du noyau aromatique qui est obtenue de préférence,
  . hydrocarbures aromatiques polycycliques (méthylnaphtalènes par exemple);

- toutes les réactions gaz-liquide photochimiques d'halogénation d'alkylbenzènes pour lesquelles des gains importants de cinétique réactionnelle et de sélectivité sont obtenus par la mise en oeuvre d'une recirculation optimisée avec la vitesse du liquide dans la zone réactionnelle, la fraction volumique du gaz dans la zone réactionnelle, le diamètre des bulles et la recirculation. Le présent réacteur permet donc de mettre en oeuvre toute réaction gaz-liquide en choisissant les valeurs optimales associées des trois paramètres ci-dessus, vitesse-fraction-diamètre.

Les réacteurs utilisés dans les procédés selon la présente invention peuvent être mis en oeuvre seuls, ou couplés par exemple en série avec d'autres réacteurs, classiques ou selon la présente invention.

Ainsi, il est possible de coupler un échangeur de chaleur externe avec recirculation sur le réacteur utilisé pour la mise en oeuvre du présent procédé. L'échangeur peut recevoir une partie ou la totalité du liquide en recirculation dans le réacteur.

Le principe général du présent procédé peut être transposé à des réacteurs de conception traditionnelle; il est en effet possible de modifier avantageusement ces réacteurs en incorporant une virole intermédiaire.

A titre d'exemple de réaction photochimique mise en oeuvre par le présent procédé, on peut citer la réaction de chloration du toluène, dans laquelle on peut obtenir trois produits recherchés, le chlorure de benzyle (monosubstitution), le chlorure de benzylidène (disubstitution) et le phénylchloroforme (trisubstitution ou substitution complète).

Lorsque l'on cherche à produire essentiellement du chlorure de benzyle, c'est-à-dire selon un rapport chlorure de benzylidène/chlorure de benzyle < 0,15, on utilise généralement un seul réacteur. On peut réaliser une chloration incomplète, de façon à obtenir un mélange en sortie de réacteur contenant en moyenne:

| . toluène | 50 à 40% |
|---|---|
| . chlorure de benzyle | 46 à 53% |
| . chlorure de benzylidène | 2 à 7% |

Il est aussi possible de monter plusieurs réacteurs plus petits en série, ce qui permet d'obtenir une proportion de chlorure de benzylidène plus faible, ce qui peut être avantageux.

Lorsque l'on cherche à produire du chlorure de benzylidène, on peut opérer comme suit. L'effluent gazeux du deuxième réacteur est recyclé vers le premier réacteur de façon à le débarrasser des dernières quantités résiduelles de chlore non-fixé.

Lorsque l'on cherche à produire du phénylchloroforme, on opère généralement avec plusieurs réacteurs en série. On utilise de 2 à 6 réacteurs en série, voir plus, mais avantageusement de 3 à 5 réacteurs. La figure 8 donne un exemple d'une installation ou ligne de production de phénylchloroforme. Le premier réacteur est alimenté en toluène. Le chlore nécessaire est réparti, selon une alimentation étagée, de façon calculée entre les différents réacteurs de manière à assurer une progression régulière de la chloration du premier jusqu'au dernier réacteur. Le premier réacteur est en outre alimenté avec les effluents gazeux des réacteurs suivants, de sorte à recycler le chlore non-fixé à l'issue des réactions intermédiaires et de la réaction finale mise en oeuvre dans lesdits réacteurs suivants. Cette installation permet d'obtenir en sortie du dernier réacteur un phénylchloroforme à teneur garantie de 98%. Par ailleurs, cette installation permet de soutirer, aux autres étages, et particulièrement au premier étage, des mélanges riches en chlorure de benzyle et/ou chlorure de benzylidène. On obtient après distillation de ces mélanges du chlorure de benzyle ou du chlorure de benzylidène de haute pureté. Grâce au réacteur selon la présente invention, il est en outre possible de réduire le nombre de réacteurs en série dans une ligne de production, notamment de phénylchloroforme.

Il est ainsi possible, grâce aux avantages auxquels conduit le présent réacteur, d'atteindre des capacités de production de 20 à 25 000 tonnes/an, exprimées en chlore fixé, soit dans un réacteur unique s'il s'agit de chlorure de benzyle, soit dans une ligne réduite s'il s'agit de phénylchloroforme. Par ailleurs, l'augmentation de la vitesse de chloration de la chaîne latérale du toluène et la diminution correspondante des temps de séjour, notamment de la phase liquide, se font au détriment de la chloration sur le noyau qui est reculée dans des limites inaccessibles par l'emploi de réacteurs classiques. Il est, de ce fait, possible d'obtenir du chlorure de benzyle à moins de 500 ppm de chlorotoluène ainsi que du phénylchloroforme avec une pureté supérieure à 98% en sortie de réacteur. Il est ainsi possible d'éviter la distillation coûteuse de ces produits.

Les avantages mis en évidence dans le cas de la chloration du toluène ne sont pas spécifiques à cette réaction particulière; ils sont atteints pour la généralités des réactions photochimiques d'halogénation d'alkylbenzènes.

Les exemples suivants illustrent l'invention, sans la limiter.

EXEMPLE 1 - Production de chlorure de benzyle

On utilise un réacteur cylindroconique à trois niveaux d'injection, tel que présenté figure 7. Les dimensions de ce réacteur, en référence à la figure, sont:

| $D_1 ih$ | 1,35 m |
|---|---|
| $H_1$ | 0,75 m |
| $D_2 ib$ | 0,75 m |
| $H_2$ | 0,75 m |

(suite)

| $D_1ih - D_2eh$ | 44 mm |
|---|---|

La réaction est mise en oeuvre selon les conditions opératoires suivantes:

| | |
|---|---|
| . vitesse du liquide dans la zone réactionnelle | $0,39 \text{ m.s}^{-1}$ |
| . vitesse du liquide dans la zone de recirculation | $1,82 \text{ m.s}^{-1}$ |
| . vitesse du gaz dans la zone réactionnelle | $0,61 \text{ m.s}^{-1}$ |
| . % volume de la phase gaz | 28% |
| . température (T) | 95 °C |
| . pression (P) | 760 Torr (0,1MPa) |
| . débit gazeux total sous T et P ($Cl_2$ + HCl + organiques vaporisés). | $0,184 \text{ m3.s}^{-1}$ |
| . débit de chlore | $900 \text{ kg.h}^{-1}$ |

Le régime normal de fonctionnement est établi, et on obtient les résultats suivants, établis sur une durée de production de 3 jours, après distillation du mélange sortant du réacteur.

| | Quantité (3 jours) | Pureté | Teneur en chlorotoluènes |
|---|---|---|---|
| . Chlorure de benzyle | 99,6 tonnes | 99,85 | < 500 ppm |
| . chlorure de benzylidène | 9,5 tonnes | | |

EXEMPLE 2 - Production de phénylchloroforme

On utilise une série de réacteurs dont les dimensions sont, en référence à la figure 2 (la virole s'arrête à un niveau intermédiaire entre l'unique distributeur et le pied du réacteur), les suivantes:

| | |
|---|---|
| $D_{1i}$ | 0,99 m |
| $D_{2e}$ | 0,91 m |
| $D_{2e}$ | 0,904 m |
| $D_{3e}$ | 0,125 m |
| H | 0,9 m |

Ces réacteurs sont montés selon le schéma donné à la figure 8. L'ensemble est constitué de trois réacteurs (1, 2, 3) continus montés en série et un stade final discontinu constitué par deux réacteurs alternés (4A, 4B) dont un en service. Le chlore est réparti entre les réacteurs 1, 2, 3, 4A et 4B.

Les conditions opératoires sont indiquées dans le tableau qui suit:

| | Réacteur 1 | Réacteur 2 | Réacteur 3 |
|---|---|---|---|
| . vitesse U du liquide dans la zone réactionnelle (m/s) | 0.55 | 0,33 | 0,30 |

(suite)

|  | Réacteur 1 | Réacteur 2 | Réacteur 3 |
|---|---|---|---|
| . % phase gaz dans la zone réactionnelle (%). | 29 | 15 | 13 |
| . vitesse U + V du gaz dans la zone réactionnelle (m/s) | 0,9 | 0,8 | 0,8 |
| . température (°C) | 100 | 145 | 155 |
| . pression (torr/MPa) | 760(0,1MPA) | 850(0,112MPa) | 850(0,112MPa) |
| . débit gazeux total (m3/s) | 0,16 | 0,07 | 0,05 |
| . débit de chlore frais (kg/h) | - | 410 | 300 |
| . débit de chlore récupéré (kg/h) | 40 | - | - |

A ce stade de la chloration, la teneur en phénylchloroforme dépasse 90%, c'est-à-dire que la quantité de chlore fixé dépasse 95% du total.

Dans cet exemple particulier, le stade final est discontinu. Le dernier réacteur remplit les fonctions multiples d'ajustage de la fin de chloration mais aussi de réservoir d'attente de contrôles analytiques complémentaires. Ses dimensions sont données ci-dessous:

| $D_{1i}$ | 1,58 m |
|---|---|
| $D_{2e}$ | 1,48 m |
| H (virole intérieure) | 1,5 m |
| Volume du réacteur | 5 m3 |

Le niveau de liquide dans le dernier réacteur discontinu étant variable, la virole délimitant la zone de recirculation est munie de fentes calibrées, conformément à la figure 9, permettant d'amorcer la recirculation quel que soit le niveau de remplissage du réacteur discontinu.

Les conditions opératoires sont les suivantes:

| Température (°C) | 155 |
|---|---|
| Pression (torr/MPa) | 850(0,112MPa) |
| Débit moyen de $Cl_2$ (kg/h) | 54 |

L'effluent gazeux contenant le chlore en excès est recyclé vers le réacteur 1, conformément à la figure 8.

En régime normal de fonctionnement, on obtient les résultats:

Phénylchloroforme:

| . quantité sur 3 jours | 50,03 tonnes |
|---|---|
| . pureté | 98,41% |

Le dernier réacteur peut avantageusement être remplacé par un réacteur continu dont la virole, délimitant la zone de recirculation, descend jusqu'au fond du réacteur, conformément à la figure 3, et dont les dimensions respectives sont:

| | |
|---|---|
| $D_{1i}$ | 0,4 m |
| $D_{2e}$ | 0,3 m |
| $D_{3e}$ | 0,175 m |
| H | 1,2 m |

## Revendications

1. Procédé d'halogénation sélective d'alkylbenzènes sur la(les) chaîne(s) alkyle(s), caractérisé en ce qu'il est mis en oeuvre dans un réacteur gaz/liquide photochimique (1) à symétrie axiale, comprenant:

   - une enceinte (2);
   - une source centrale de rayonnement (3);
   - au moins un distributeur de gaz (4), en pied dudit réacteur;
   - une virole (5) disposée concentriquement entre la source de rayonnement (3) et l'enceinte (2), ladite virole permettant une recirculation interne du liquide réactionnel, selon un mouvement ascendant dans l'espace situé entre la source (3) et la virole (5), et selon un mouvement descendant dans l'espace situé entre l'enceinte (2) et la virole (5).

2. Procédé selon la revendication 1, lequel procédé étant un procédé de chloration du toluène.

3. Procédé selon la revendication 1 ou 2, caractérisé en ce qu'il est mis en oeuvre dans un réacteur dans lequel la virole (5) s'étend jusqu'au niveau du distributeur ou jusqu'à un niveau intermédiaire situé entre le distributeur (4) et le pied de l'enceinte (2).

4. Procédé selon la revendication 1 ou 2, caractérisé en ce qu'il est mis en oeuvre dans un réacteur dans lequel la virole (5) s'étend jusqu'au pied de l'enceinte (2) et comporte une série d'orifices (7) situés à un niveau intermédiaire entre le distributeur (4) et le pied de l'enceinte (2).

5. Procédé selon l'une quelconque des revendications 1 à 4, caractérisé en ce qu'il est mis en oeuvre dans un réacteur dans lequel la virole (5) comporte sur une partie ou toute sa longueur des fentes calibrées (5a).

6. Procédé selon l'une quelconque des revendications 1 à 5, caractérisé en ce qu'il est mis en oeuvre dans un réacteur dans lequel la recirculation interne commence à un niveau situé sous le distributeur (4) dans une région située en pied de l'enceinte (2).

7. Procédé selon l'une quelconque des revendications 1 à 6, caractérisé en ce qu'il est mis en oeuvre dans un réacteur dans lequel la vitesse du liquide U selon le mouvement ascendant dans l'espace situé entre la source (3) et la virole (5) est comprise entre 0,1 et 0,8 m/s.

8. Procédé selon l'une quelconque des revendications 1 à 7, caractérisé en ce qu'il est mis en oeuvre dans un réacteur dans lequel la vitesse du liquide U selon le mouvement ascendant dans l'espace situé entre la source (3) et la virole (5) est comprise entre 0,25 et 0,60 m/s.

9. Procédé selon l'une quelconque des revendications 1 à 8, caractérisé en ce qu'il est mis en oeuvre dans un réacteur dans lequel la vitesse du gaz U + V est comprise entre 0,3 et 1,1 $m.s^{-1}$.

10. Procédé selon l'une quelconque des revendications 1 à 9, caractérisé en ce qu'il est mis en oeuvre dans un réacteur dans lequel la vitesse du liquide selon le mouvement descendant dans l'espace situé entre la virole (5) et l'enceinte (2) est comprise entre 0,5 et 3 m/s.

11. Procédé selon l'une quelconque des revendications 1 à 10, caractérisé en ce qu'il est mis en oeuvre dans un réacteur dans lequel la vitesse du liquide selon le mouvement descendant dans l'espace situé entre la virole (5) et l'enceinte (2) est comprise entre l et 2,5 m/s.

**12.** Procédé selon l'une quelconque des revendications 1 à 11, caractérisé en ce qu'il est mis en oeuvre dans un réacteur dans lequel la fraction de volume occupé par la phase gaz représente de 10 à 50% du volume compris entre la source (3) et la virole (5).

**13.** Procédé selon l'une quelconque des revendications 1 à 12, caractérisé en ce qu'il est mis en oeuvre dans un réacteur dans lequel la fraction de volume occupé par la phase gaz représente de 20 à 40% en volume du volume compris entre la source (3) et la virole (5).

**14.** Procédé selon l'une quelconque des revendications 1 à 13, caractérisé en ce qu'il est mis en oeuvre dans un réacteur dans lequel le diamètre des bulles générées par le distributeur (4) est compris entre 3 et 15 mm.

**15.** Procédé selon l'une quelconque des revendications 1 à 14, caractérisé en ce qu'il est mis en oeuvre dans un réacteur dans lequel le diamètre des bulles générées par le distributeur (4) est compris entre 6 et 10 mm.

**Claims**

**1.** A process for selectively halogenating alkylbenzenes on the alkyl chain or chains, characterized in that said process is carried out in an axially symmetrical photochemical gas/liquid reactor (1), comprising:

- a vessel (2);
- a central source of radiation (3);
- at least one distributor for gas (4), at the bottom of said reactor;
- inner sleeve means (5) arranged concentrically between the source of radiation (3) and the vessel (2), said inner sleeve means allowing internal recirculation of reaction liquid, said recirculation comprising a rising movement in the space situated between the source (3) and the inner sleeve means (5) and a descending movement in the space situated between the vessel (2) and the inner sleeve means (5).

**2.** The process according to claim 1 characterized in that said process is a process for chlorination of toluene.

**3.** The process according to claim 1 or 2, characterized in that said process is carried out in a reactor in which the inner sleeve means (5) extends down to the level of the distributor or down to an intermediate level situated between the distributor (4) and the bottom of the vessel (2).

**4.** The process according to claim 1 or 2, characterized in that said process is carried out in a reactor in which the inner sleeve means (5) extend down to the bottom of the vessel (2) and includes a series of orifices (7) located at an intermediate level between the distributor (4) and the bottom of the vessel (2).

**5.** The process according to any one of claims 1 to 4, characterized in that said process is carried out in a reactor in which the inner sleeve means (5) includes slots of a calibrated size (5a) along part or all of its length.

**6.** The process according to any one of claims 1 to 5, characterized in that said process is carried out in a reactor in which internal recirculation starts at a point below the distributor (4), in the vicinity of the bottom of said reactor (2).

**7.** The process according to any one of claims 1 to 6, characterized in that said process is carried out in a reactor in which the velocity U of the liquid in the rising movement occurring in the space situated between said source (3) and said inner sleeve member (5) is comprised between 0.1 and 0.8 m/s

**8.** The process according to any one of claims 1 to 7, characterized in that said process is carried out in a reactor in which the velocity U of the liquid in the rising movement occurring in the space situated between said source (3) and said inner sleeve member (5) is comprised between 0.25 and 0.60 m/s.

**9.** The process according to any one of claims 1 to 8, characterized in that said process is carried out in a reactor in which the velocity of the gas U + V is comprised between 0.3 and 1.1 m/s.

**10.** The process according to any one of claims 1 to 9, characterized in that said process is carried out in a reactor in which the velocity of the liquid in said descending movement situated in the space between said inner sleeve means (5) and said vessel (2) is comprised between 0.5 and 3 m/s.

11. The process according to any one of claims 1 to 10, characterized in that it is carried out in a reactor in which the velocity of the liquid in said descending movement situated in the space between said inner sleeve means (5) and said vessel (2) is comprised between 1 and 2.5 m/s.

12. The process according to any one of claims 1 to 11, characterized in that it is carried out in a reactor in which the fraction of the volume occupied by the gaseous phase represents 10 to 50% of the volume of said vessel defined between said source (3) and said inner sleeve means (5).

13. The process according to any one of claims 1 to 12, characterized in that it is carried out in a reactor in which the fraction of the volume occupied by the gaseous phase represents 20 to 40% by volume, of the volume of said vessel defined between said source (3) and said inner sleeve means (5).

14. The process according any one of claims 1 to 13, characterized in that said process is carried out in a reactor in which the diameter of the bubbles generated by the said distributor is comprised between 3 and 15 mm.

15. The process according any one of claims 1 to 14, characterized in that said process is carried out in a reactor in which the diameter of the bubbles generated by the said distributor (4) is comprised between 6 and 10 mm.

**Patentansprüche**

1. Selektives Halogenierungsverfahrenvon Alkylbenzolen an deren Alkylkette(n), gekennzeichnet dadurch, daß es in einem photochemischen Gas/Flüssig-Reaktor (1) mit Axialsymmetrie durchgeführt wird, wobei dieser umfaßt:

   - einen Behälter (2);
   - eine zentrale Strahlungsquelle (3);
   - mindestens einen Gasverteiler (4) am Fuße des Reaktors;
   - ein konzentrisch zwischen der Strahlungsquelle (3) und dem Behälter (2) angeordneter Mantel (5), wobei der Mantel den internen Umlauf der Reaktionsflüssigkeit in aufsteigender Richtung in dem von der Quelle (3) und dem Mantel (5) begrenzten Raum und in absteigender Richtung in dem von dem Behälter (2) und dem Mantel (5) begrenzten Raum erlaubt.

2. Verfahren gemäß Anspruch 1, wobei dieses Verfahren ein Chlorierungsverfahren von Toluol ist.

3. Verfahren gemäß Anspruch 1 oder 2, gekennzeichnet dadurch, daß es in einem Reaktor durchgeführt wird, in dem der Mantel (5) sich bis zur Höhe des Gasverteilers erstreckt oder bis zu einer mittleren Höhe, die zwischen dem Gasverteiler (4 ) und dem Fuß des Behälters (2) liegt.

4. Verfahren gemäß Anspruch 1 oder 2, gekennzeichnet dadurch, daß es in einem Reaktor durchgeführt wird, in dem der Mantel (5) sich bis zum Fuß des Behälters (2) erstreckt und in mittlerer Höhe zwischen dem Verteiler (4) und dem Fuß des Behälters (2) eine Reihe von Öffnungen aufweist.

5. Verfahren gemäß mindestens einem der Ansprüche 1 bis 4, gekennzeichnet dadurch, daß es in einem Reaktor durchgeführt wird, in dem der Mantel (5) auf einem Teil oder auf seiner ganzen Länge kalibrierte Schlitze (5a) aufweist.

6. Verfahren gemäß mindestens einem der Ansprüche 1 bis 5, gekennzeichnet dadurch, daß es in einem Reaktor durchgeführt wird, in dem der interne Umlauf auf einer Höhe beginnt, die unter dem Verteiler (4) liegt, in einem am Fuße des Behälters (2) befindlichen Bereich.

7. Verfahren gemäß mindestens einem der Ansprüche 1 bis 6, gekennzeichnet dadurch, daß es in einem Reaktor durchgeführt wird, in dem die Geschwindigkeit der Flüssigkeit U in aufsteigender Richtung im von der Quelle (3) und dem Mantel (5) begrenzten Raum zwischen 0,1 und 0,8 m/s liegt.

8. Verfahren gemäß mindestens einem der Ansprüche 1 bis 7, gekennzeichnet dadurch, daß es in einem Reaktor durchgeführt wird, in dem die Geschwindigkeit der Flüssigkeit U in aufsteigender Richtung im von der Quelle (3) und dem Mantel (5) begrenzten Raum zwischen 0,25 und 0,6 m/s liegt.

9. Verfahren gemäß mindestens einem der Ansprüche 1 bis 8, gekennzeichnet dadurch, daß es in einem Reaktor durchgeführt wird, in dem die Geschwindigkeit des Gases U + V zwischen 0,3 und 1,1 m/s liegt.

10. Verfahren gemäß mindestens einem der Ansprüche 1 bis 9, gekennzeichnet dadurch, daß es in einem Reaktor durchgeführt wird, in dem die Geschwindigkeit der Flüssigkeit in absteigender Richtung im von dem Mantel (5) und dem Behälter (2) begrenzten Raum zwischen 0,5 und 3 m/s liegt.

11. Verfahren gemäß mindestens einem der Ansprüche 1 bis 10, gekennzeichnet dadurch, daß es in einem Reaktor durchgeführt wird, in dem die Geschwindigkeit der Flüssigkeit in absteigender Richtung im von dem Mantel (5) und dem Behälter (2) begrenzten Raum zwischen 1 und 2,5 m/s liegt.

12. Verfahren gemäß mindestens einem der Ansprüche 1 bis 11, gekennzeichnet dadurch, daß es in einem Reaktor durchgeführt wird, in dem der von der Gasphase eingenommene Volumenanteil 10 bis 50 % des von der Quelle (3) und dem Mantel (5) begrenzten Volumens ausmacht.

13. Verfahren gemäß mindestens einem der Ansprüche 1 bis 12, gekennzeichnet dadurch, daß es in einem Reaktor durchgeführt wird, in dem der von der Gasphase eingenommene Volumenanteil 20 bis 40 % des von der Quelle (3) und dem Mantel (5) begrenzten Volumens ausmacht.

14. Verfahren gemäß mindestens einem der Ansprüche 1 bis 13, gekennzeichnet dadurch, daß es in einem Reaktor durchgeführt wird, in dem der Durchmesser der vom Verteiler (4) gebildeten Blasen zwischen 3 und 15 mm liegt.

15. Verfahren gemäß mindestens einem der Ansprüche 1 bis 14, gekennzeichnet dadurch, daß es in einem Reaktor durchgeführt wird, in dem der Durchmesser der vom Verteiler (4) gebildeten Blasen zwischen 6 und 10 mm liegt.

FIG.1

FIG.9

FIG.2

FIG.3

FIG.4

FIG.5

FIG.6a

FIG.6b

FIG.7

FIG.8

16